# EUROPÄISCHE PATENTSCHRIFT

(11) **EP 1 601 389 B1**
(45) Veröffentlichungstag und Bekanntmachung des Hinweises auf die Patenterteilung: **21.06.2006**
(21) Anmeldenummer: 04717646.6
(22) Anmeldetag: 05.03.2004
(51) Int. Cl.: A61L 27/04, A61L 31/02

(54) **VERWENDUNG VON AUSSCHEIDUNGSHÄRTBAREM, MARTENSITISCHEM, ROST FREIEM STAHL**
USE OF MARTENSITIC PRECIPITATION HARDENING STAINLESS STEEL
UTILISATION D'ACIER INOXYDABLE MARTENSITIQUE, DURCISSABLE PAR PRECIPITATION

(30) Priorität: 07.03.2003 SE 0300644
(43) Veröffentlichungstag der Anmeldung: 07.12.2005
(73) Patentinhaber: Sandvik Intellectual Property AB, 811 81 Sandviken (SE)
(72) Erfinder: BLANKE, Bernd, 77736 Zeil a.H. (DE); GÖKEN, Matthias, 49808 Lingen (Ems) (DE)
(74) Vertreter: Weber, Dieter
(86) Internationale Anmeldenummer: PCT/EP2004/050265
(87) Internationale Veröffentlichungsnummer: WO 2004/078224

(56) Entgegenhaltungen:
- WO-A-01/14601
- WO-A-01/31076
- WO-A-93/07303
- WO-A-02/078763
- WO-A-02/078764
- US-A- 3 918 101
- DATABASE WPI Section PQ, Week 200019 Derwent Publications Ltd., London, GB; Class P31, AN 2000-221366 XP002289002 & RU 2 122 370 C1 (URALS TRAVMATOLOGY ORTHOPAEDICS RES INST) 27. November 1998 (1998-11-27)
- DATABASE WPI Section Ch, Week 200048 Derwent Publications Ltd., London, GB; Class D22, AN 2000-530696 XP002289003 & RU 2 143 248 C1 (PIDAR YU P) 27. Dezember 1999 (1999-12-27)

## Beschreibung

### Technisches Umfeld

Die Erfindung betrifft die neuartige Verwendung von auscheidungshärtbaren, martensitischen, rostfreien Stählen, insbesondere deren Verwendung in medizinischen Anwendungen, wie Implantaten und Osteosyntheseprodukten zur Anwendung und zum Verbleib im oder am menschlichen Körper.

### Hintergrund und technische Problemstellung

Ausscheidundshärtbare, martensitische, rostfreie Stähle sind bekannt durch WO 93/07303. Dort wird eine Zusammensetzung eines rostfreien Stahls beschrieben, der eine sehr hohe Festigkeit bei gleichzeitiger guter Duktilität aufweist. Dieser Stahl wird als besonders geeignet für die Herstellung von Injektionskanülen, zahnärztlichen Instrumenten und medizinischen Instrumenten beschrieben.
In WO 01/14601 A1 wird ein Verfahren zur Herstellung von medizinischen oder zahnärztlichen Instrumenten durch eine Reihe von Verfahrensschritten einschliesslich Ausscheidungshärtung, Tempern, Abschrecken und Härten beschrieben, dessen Ergebnis eine homogene Härte von mindestens 450 HV darstellt.
Es wird beispielhaft spezifiziert, dass ein ausscheidungshärtbarer, martensitischer, rostfreier Stahl zur Herstellung von medizinischen Instrumenten mit dem dort spezifizierten Prozess verwendet werden kann.

Für eine spezifische Anwedung eines Stahls in medizinischen Anwendungen, wie Implantaten und Osteosyntheseprodukten gibt es eine Reihe von Randbedingungen, die erfüllt werden sollten.
Implantate und Osteosyntheseprodukte, wie Platten, Befestigungselemente, wie Schrauben und Nägel, haben je nach Anwendungszweck sehr ungünstige Geometrien, d.h. zum Beispiel ein sehr ungünstiges Verhältnis von Länge zu Dicke und/oder Durchmesser. Aufgrund ihrer ungünstigen Geometrie sind solche Implantate und Osteosyntheseprodukte sehr empfindlich gegenüber den im praktischen Gebrauch oft leichten oder stärker anliegenden Biegebelastungen. Bereits ein leichtes, kaum feststellbares Verbiegen Implantate und/oder Osteosyntheseprodukte führt dazu, dass sie bei der nächsten Belastung brechen können. Aufgrund der häufig sehr hohen Biegebelastungen führt dies in der Praxis gelegentlich dazu, dass die Implantate und Osteosyntheseprodukte brechen. Dies bedeutet nicht nur, dass sie eine kurze Lebensdauer haben, sondern auch eine erhebliche Verletzungsgefahr für den Patienten.
Es besteht daher ein dringender Bedarf nach implantaten und Osteosyntheseprodukten, die eine hohe Härte besitzen, korrosionsbeständig und gleichzeitig auch gegenüber bekannten Implantaten und Osteosyntheseprodukten bruchsicher sind. Neben Härte und Bruchsicherheit ist die gleichzeitige Korrosionsbeständigkeit und Biokompatibilität von entscheidender Bedeutung. Die korrosiven Medien beim Gebrauch wie z.B. Blut und andere Körperflüssigkeiten. Werden also solche Implantate und Osteosyntheseprodukte durch Korrosion beschädigt bzw. angegriffen, besteht die Gefahr, dass Patienten mit den Korrosionsrückständen kontaminiert werden und gefährliche postoperative Komplikationen entstehen.

Eine andere typische Anwendung findet sich in der Chirurgie. Dort werden häufig metallische Platten, Nägel und Schrauben, sogenannte Osteosyntheseprodukte, zum Verbinden von Knochen nach Brüchen oder nach operativem Zersägen der Knochen eingesetzt, d. h. implantiert. Diese Implantate verbleiben dann in der Regel bis zur vollständigen Heilung des Knochens und darüber hinaus im Körper des Patienten. Bekannte, auf dem Markt erhältliche Osteosyntheseprodukte sind aus sogenanntem rostfreiem Stahl, wie den oben genannten Stahlqualitäten, hergestellt. Da für solche Osteosyntheseprodukte zusätzlich zu den mechanischen und Korrosionseigenschaften auch noch hohe Anforderungen an die Biokompatibilität gestellt werden, steht nur eine begrenzte Auswahl an Materialien für die Herstellung dieser Produkte zur Verfügung (nicht alle in der unten aufgeführten Tabelle 1 genannten Werkstoffe erfüllen diese Bedingung). Es wurde festgestellt, dass die Bruchfestigkeit der bekannten Implantate, insbesondere nach längerer Verwendungsdauer, schlecht ist. Aus diesem Grund sind Patienten angehalten, mit solchen Osteosyntheseprodukten verbundene Knochen nach der Operation zunächst zu schonen und nur schwach zu belasten. Um eine Rückbildung der unterbelasteten Muskulatur zu verhindern, ist es notwendig so bald als möglich mit Hilfe der Krankengymnastik einen Abbau zu verlangsamen, denn verhindern läßt er sich nicht. Hält sich der Patient nicht an die vorgeschriebene Schonung des operierten Knochens oder aufgrund ungeschickter Bewegungen, kann es zu Biegebelastungen der Osteosyntheseprodukte bzw. Implantate und schließlich zu deren Bruch kommen. Hierdurch geht nicht nur die durch das Anbringen des Implantats gewünschte Verbindung der Knochenfragmente verloren, das oft scharfkantig brechende Implantat kann auch erhebliche Verletzungen des Patienten hervorrufen. In jedem Fall wird bei einem Bruch des Implantats eine zusätzliche Operation des Patienten zur Behebung des Schadens erforderlich. Durch zu geringe Festigkeit der Produkte besteht zusätzlich die Gefahr, dass sich verschraubte Platten durch Mikroschwingungen lockern und so die Knochenheilung hinauszögen.
Es besteht daher ein dringender Bedarf nach stabilen, korrosionsbeständigen und biokompatiblen Osteosyntheseprodukten bzw. Implantaten, die gleichzeitig hohe Festigkeitswerte verbunden mit einer guten Duktilitätseigenschaften bieten.
Derzeit wird eine Reihe gut bekannter und gut untersuchter Legierungstypen zum Formen und Herstellen solcher Werkzeuge und Implantate verwendet. Einige dieser Legierungen sind martensitische nicht rostende Stähle, austenitische nicht rostende Stähle, und ausscheidungshärtbare nicht rostende Stähle. Jede dieser bekannten Legierungen weist eine Reihe von guten Materialeigenschaften auf, wie Korrosionsbeständigkeit, Festigkeit, Formbarkeit und/oder Duktilität, doch hat jede Legierung auch Nachteile und kann bestimmten Produktanforderungen nicht entsprechen. Aus der Praxis sind sind komplexe Probleme und Nachteile von derzeit auf dem Markt erhältlichen Rotationswerkzeugen, chirurgischen Implantaten, chirurgischen Osteosyntheseprodukten, wie chirurgischen Platten, Schrauben und Nägeln, bekannt.

Die nachfolgende Tabelle zeigt die Zusammensetzungen einiger häufig verwendeter Stähle.

**Tabelle 1: Zusammensetzungen verschiedener bekannter Stähle in Gew.-%; Rest Eisen**

| Legierung | C | Si | Mn | S | Cr | Ni | Mo | Cu | Ti | N | P |
|---|---|---|---|---|---|---|---|---|---|---|---|
| AISI 420 | 0,36 | 0.15 | 0,30 | <0,020 | 13,5 | <0,3 | | | | | |
| AISI 420F | 0,22 | 0,58 | 1,58 | 0,175 | 13,0 | 0,8 | 1,2 | | | | |
| AISI 304 | 0,060 | 0,66 | 1,22 | 0,002 | 18,6 | 8,6 | 0,2 | | | | |
| ISO 5832-1-D | <0,03 | <1,0 | <2,0 | <0,01 | 17,5 | 14 | 2,8 | <0.5 | | <0,1 | <0,025 |
| ISO 5832-9 | 0,08 | <0,75 | 3,6 | <0,01 | 20,5 | 10,0 | 2,5 | <0,25 | | 0,4 | <0,025 |
| Carpenter 455 | 0,006 | 0,07 | 0,03 | 0,004 | 11,4 | 8,3 | <0,1 | 2,2 | 1,2 | | |
| C455 (V) | 0,004 | 0,04 | 0,15 | 0,002 | 11,8 | 9,1 | <0,1 | 2,0 | 1,6 | | |
| 1.4108 | 0,31 | 0,68 | 0,41 | 0,002 | 15,54 | 0,16 | 0,97 | | | 0,41 | 0,017 |
| 1.4112 | 0,85-0,95 | <1,0 | <1,0 | 0,030 | 17,0-19,0 | | 0,9-1,3 | | | | 0,040 |

Martensitische, nicht rostende Stähle, z.B. die Qualitäten AISI 420, können eine hohe Festigkeit bieten, doch nicht in Kombination mit Duktilität. Austenitische, nicht rostende Stähle, z.B. die Reihe AISI 300, können gute Korrosionsbeständigkeit in Verbindung mit hoher Festigkeit und für einige Anwendungen annehmbarer Duktilität bieten, doch ist, um die fiohe Festigkeit zu erreichen, eine starke Kaltreduktion erforderlich, und dies bedeutet, dass auch das halbfertige Produkt eine sehr hohe Festigkeit haben muss, was wiederum eine schlechte Formbarkeit zur Folge hat. Für die Gruppe der ausscheidungshärtbaren, nicht rostenden Stähle, gibt es zahlreiche unterschiedliche Qualitäten und alle mit unterschiedlichen Eigenschaften. Sie haben jedoch einige Gemeinsamkeiten, beispielsweise werden die meisten von ihnen in einem Einweg- oder üblicher in einem Zweiwegverfahren in Vakuum geschmolzen, wobei die zweite Stufe ein Aufschmelzen unter Vakuum ist. Außerdem ist eine große Menge von ausscheidungsbildenden Elementen, wie Aluminium, Niob, Tantal und Titan, erforderlich und oftmals als Kombinationen dieser Elemente. Unter "groß" versteht man > 1,5%. Eine große Menge ist günstig für die Festigkeit, vermindert aber die Duktilität und die Formbarkeit. Eine Qualität findet sich z. B. in der US-Patentschrift 3 408 871. Diese Qualität bietet eine annehmbare Duktilität in dem Fertigprodukt, doch in Verbindung mit einer Festigkeit von nur etwa 2.000 N/mm². Sie kann auch während der Herstellung von halbfertigen Produkten Nachteile haben, z.B. ist der Stahl empfindlich für Rissbildung im geglühten Zustand.

### Beschreibung der Erfindung

Ein Aspekt der Erfindung betrifft die von ausscheidungshärtbarem, martensitischem, rostfreiem Stahl in der unten genannten Zusammensetzung für die Herstellung von chirurgischen Implantaten, chirurgischen Oestesyntheseprodukten, chirurgischen Platten, Schrauben und Nägeln zur Anwendung und zum Verbleib im oder am menschlichen Körper.
Ein überraschender, von der Erfindung erfasster Effekt ist, dass auscheidungshärtbarer, martensitischer, rostfreier Stahl in solchen Anwendungen von Vorteil ist, in denen die Kombination von hoher Bruch- und Biegefestigkeit mit Härte- und Korrosionseigenschaften eine entscheidende Rolle spielt.
Ein weiterer überraschender, von der Erfindung erfasster Effekt betrifft die vorteilhafte Kombination von guter biologischer Verträglichkeit des unter bschriebenen Stahls mit guten Korrosionseigenschaften, hoher Duktilität, guter Formbarkeit und ausgezeichnet hohen Festigkeit von etwa 2.500 bis 3.000 N/mm2. Diese Kombination erlaubt die vorteilhafte Nutzung dieses Stahls in medizinischen Anwendungen, in denen das Material für einen kürzeren oder längeren Zeitraum im Körper des Patienten verbleibt.

Der in der Erfindung beschriebene ausscheidungshärtbare, martensitische, rostfreie Stahl soll die nachfolgend beschriebene Zusammensetzung besitzen:

| | |
|---|---|
| Chrom | 10 bis 14 |
| Nickel | 7 bis 11 |
| Molybdän | 0,5 bis 6 |
| Kupfer | 0,5 bis 4 |
| Aluminium | 0,05 bis 0,55 |
| Titan | 0,4 bis 1,4 |
| Kohlenstoff + Stickstoff | bis zu 0,3 |
| Schwefel | weniger als 0,05 |
| Phosphor | weniger als 0,05 |
| Mangan | bis zu 0,5 |
| Silizium | bis zu 0,5 |
| Tantal, Niob, Vanadium und Wolfram | jeweils bis zu 0,2 |
| Kobalt | gegebenenfalls bis zu 9,0 |
| Bor | gegebenenfalls 0,0001 bis 0,1 |

wobei der Rest aus Eisen und üblichen Verunreinigungen besteht.

### Beispiele zur Beschreibung der erfindungsgemässen Eigenschaften

Zugfestigkeit, Bruchdehnung und Härte wurden an ausgehärteten Vollmaterialwerkstücken gleicher Geometrie aus dem erfindungsgemäß verwendeten Stahl und zwei derzeit für Rotationswerkzeuge eingesetzten Stählen getestet.

Bei dem getesteten erfindungsgemäßen Stahl handelt es sich um eine Zusammensetzung gemäß der oben genannten bevorzugten Ausführungsform mit 12,0 Gew.-% Chrom, 9,1 Gew.-% Nickel, 4,0 Gew.-% Molybdän, 2,0 Gew.-% Kupfer, 0,9 Gew.-% Titan, 0,35 Gew.-% Aluminium, <0,012 Gew.-% Kohlenstoff und <0,012 Gew.-% Stickstoff. Als Vergleichsstähle wurden die Qualitäten 1.4112 und 1.4108 verwendet, deren Zusammensetzungen oben in Tabelle 1 angegeben sind. Die Proben waren Vollmaterialstangen mit kreisförmigem Querschnitt und einem Durchmesser von 4,5 mm. Alle getesteten Proben waren ausscheidungsgehärtet. Die Aushärtung des erfindungsgemässen Stahls erfolgte bei 475°C für 4 Stunden. Die Aushärtung der Qualitäten 1.4112 und 1.4108 erfolgte nach den für diese Stähle vorgeschriebenen Aushärtungsverfahren. Die Aushärtung der Qualitäten 1.4112 und 1.4108 erfolgt bei 1000°C für 40-60 Minuten im Vakuum. Anschließend werden beide Qualitäten in Stickstoff auf 50°C abgekühlt. Der Werkstoff 1.4108 wurde noch zusätzlich bei 160°C für 2 Stunden angelasen. Die Aushärtung der jeweiligen Materialien wurde so durchgeführt, dass für alle getesteten Materialien eine vergleichbare Materialhärte erzielt wurde. Es wurden jeweils vier Proben eines Materials getestet. Die Ergebnisse sind in der nachfolgenden Tabelle 2 zusammengefaßt.

**Tabelle 2 Versuchsergebnisse, Zugversuch nach DIN EN 10002-1**

| **Werkstoff** | **Zugfestigkeit [MPa]** | **Bruchdehnung [%]** | **Rockwell C-Härte** |
|---|---|---|---|
| Erfindungsgemäss A | 1935 | 9,1 1 | 52/53 |
| Erfindungsgemäss A | 1938 | 9,1 1 | 52/53 |
| Erfindungsgemäss C | 1941 | 9,1 | 52/53 |
| Erfind ungsgemäss D | 1946 | 9,1 | 52/53 |
| 1.4112 | 1989 | <2 | 54/55 |
| 1.4112 | 1981 | <2 | 54/55 |
| 1.4112 | 1987 | <2 | 54/55 |
| 1.4112 | 2000 | <2 | 54/55 |
| 1.4108 | 1323 | <2 | 54/55 |
| 1.4108 | 1263 | <2 | 54/55 |
| 1.4108 | 1153 | <2 | 54/55 |
| 1.4108 | 1312 | <2 | 54/55 |

Eine Untersuchung der Bruchstellen der vorgenannten getesteten Werkstoffe zeigte deutlich, dass der erfindungsgemässe Stahl ein äußerst zähes Bruchverhalten aufwies. Die Bruchflächen hatten die Form eines sogenannten "Trichterbruchs". Demgegenüber zeigten die Werkstoffe 1.4112 und 1.4108 sogenannte Spaltbrüche mit einem nahezu 100%-igen Sprödbruchanteil. Das gute Bruchdehnungsverhalten der Proben aus dem erfindungsgemässen Stahl geht einher mit einer hohen Biegsamkeit, ohne dass das Material bricht. Die Proben können ohne Bruch mehrfach gebogen werden. Demgegenüber zerbrachen die Proben aus den Werkstoffen 1.4112 und 1.4108 beim ersten Verbiegen.

Es hat sich überraschend gezeigt, dass die Verwendung der erfindungsgemäß eingesetzten Stahlsorte zur Herstellung von Implantaten und Osteosyntheseprodukten, wie chirurgischen Platten, Nägeln und Schrauben zur Anwendung und zum Verbleib im oder am menschlichen Körper, besondere Vorteile aufgrund des hervorragenden Bruchdehnungsverhaltens der Stahlqualität gegenüber bisher verwendeten Stählen bietet. Bei den bisher verwendeten Stählen standen insbesondere Härte und Korrosionsfestigkeit sowie je nach Anwendung auch Biokompatibilität im Vordergrund, und hinsichtlich der Bruchfestigkeit wurde ein Kompromiß in Kauf genommen. Durch die erfindungsgemäße Verwendung des vorliegenden Stahls zur Herstellung von Rotationswerkzeugen und Osteosyntheseprodukten konnten nun auch die Nachteile aus dem Bruchverhalten der bisher auf dem Markt erhältlichen Produkte überwunden werden. Die erfindungsgemäß hergestellten Werkzeuge und Osteosyntheseprodukte vereinen Härte, höchste Korrosionsbeständigkeit, gute Biokompatibilität und hervorragende Bruchfestigkeit in den hergestellten Produkten. Die Produkte bleiben auch beim Verbiegen bruchsicher und können, wie zum Beispiel bei Implantaten der plastischen Chirurgie, mehrfach gebogen werden, ohne ihre hervorragenden Materialeigenschaften einzubüßen.
Darüber hinaus sind die erfindungsgemäß verwendeten Stahlqualitäten gut zerspanbar und in gehärtetem Zustand gut fräsbar, was Vorteile bei der Herstellung der Produkte liefert. Ein weiterer Vorteil der Verwendung des erfindungsgemäß eingesetzten Stahls zur Herstellung von Implantaten und Osteosyntheseprodukten zur Anwendung und zum Verbleib im oder am menschlichen Körper ist die verhältnismäßig niedrige Härtungstemperatur im Bereich von 425 bis 525°C, wodurch sich erhebliche Energiekosten bei der Herstellung einsparen lassen.

## Patentansprüche

1. Verwendung eines ausscheidungshärtbaren, martensitischen, nicht rostenden Chrom-Nickel-Stahls mit folgender Zusammensetzung (in Gewichts-%):
| | |
|---|---|
| Chrom | 10 bis 14 |
| Nickel | 7 bis 11 |
| Molybdän | 0,5 bis 6 |
| Kupfer | 0,5 bis 4 |
| Aluminium | 0,05 bis 0,55 |
| Titan | 0,4 bis 1,4 |
| Kohlenstoff + Stickstoff | bis zu 0,3 |
| Schwefel | weniger als 0,05 |
| Phosphor | weniger als 0.05 |
| Mangan | bis zu 0,5 |
| Silizium | bis zu 0,5 |
| Tantal, Niob, Vanadium und Wolfram | jeweils bis zu 0,2 |
| Kobalt | gegebenenfalls bis zu 9,0 |
| Bor | gegebenenfalls 0,0001 bis 0,1 |
wobei der Rest aus Eisen und üblichen Verunreinigungen besteht, zur Herstellung von chirurgischen Implantaten und Osteosyntheseprodukten zur Anwendung und zum Verbleib im oder am menschlichen Körper.

## Claims

1. Use of a precipitation hardenable, martensitic, stainless chrome nickel steel with the following composition (in % by weight):
| | |
|---|---|
| Chromium | 10 to 14 |
| Nickel | 7 to 11 |
| Molybdenum | 0.5 to 6 |
| Copper | 0.5 to 4 |
| Aluminium | 0.05 to 0.55 |
| Titanium | 0.4 to 1.4 |
| Carbon + nitrogen | up to 0.3 |
| Sulphur | less than 0.05 |
| Phosphorus | less than 0.05 |
| Manganese | up to 0.5 |
| Silicon | up to 0.5 |
| Tantalum, niobium, vanadium and tungsten | up to 0.2 each |
| Cobalt | up to 9.0 if applicable |
| Boron | 0.0001 to 0.1 if applicable |
with the remainder comprising iron and the normal impurities, for the manufacture of surgical implants and osteosynthesis products for application and remaining in or on the human body.

## Revendications

1. Application d'un acier au chrome-nickel, inoxydable, martensitique et durci par précipitation, dont la composition (en % en poids) est la suivante :
| | |
|---|---|
| chrome | 10 à 14 |
| nickel | 7 à 11 |
| molybdène | 0,5 à 6 |
| cuivre | 0,5 à 4 |
| aluminium | 0,05 à 0,55 |
| titane | 0,4 à 1,4 |
| carbone + azote | jusqu'à 0,3 |
| soufre | moins de 0,05 |
| phosphore | moins de 0,05 |
| manganèse | jusqu'à 0,5 |
| silicium | jusqu'à 0,5 |
| tantale, niobium, vanadium et tungstène | jusqu'à 0,2 chacun |
| cobalt | éventuellement jusqu'à 9,0 |
| bore | éventuellement de 0,0001 à 0,1 |
le reste étant du fer et des impuretés habituelles,
à la fabrication d'implants chirurgicaux et de produits d'ostéosynthèse destinés à être employés durablement dans ou sur le corps humain.
